# EUROPEAN PATENT SPECIFICATION

(11) **EP 1 337 284 B1**
(45) Date of publication and mention of the grant of the patent: **05.12.2007**
(21) Application number: 01968145.1
(22) Date of filing: 24.08.2001
(51) Int. Cl.: A61L 27/34, A61L 27/50, A61F 2/16, A61K 9/32

(54) **SWITCHABLE TACKINESS COATING COMPOSITIONS FOR OPHTHALMIC IMPLANTS**
BESCHICHTUNGSMITTEL MIT VERÄNDERBARER KLEBRIGKEIT FÜR AUGENIMPLANTATE
COMPOSITIONS DE REVETEMENT A POUVOIR ADHESIF VARIABLE POUR IMPLANTS OPHTALMIQUES

(30) Priority: 06.09.2000 US 230604 P
(43) Date of publication of application: 27.08.2003
(73) Proprietor: Alcon, Inc., 6331 Hünenberg (CH)
(72) Inventor: PATEL, Anilbhai, S., Arlington, TX 76017 (US); SCHLUETER, Douglas, C., Fort Worth, TX 76133 (US); KARAKELLE, Mutlu, Fort Worth, TX 76132 (US)
(74) Representative: Teipel, Stephan
(86) International application number: PCT/US2001/026624
(87) International publication number: WO 2002/019992

(56) References cited:
- WO-A-92/13901
- WO-A-97/06127
- WO-A-99/06495
- DE CREVOISER E.A.: "Switchable Tackiness and Wettability of a Liquid Crystalline Polymer" SCIENCE, vol. 285, 20 August 1999 (1999-08-20), pages 1246-1249, XP002202638 WASHINGTON, DC, US cited in the application

## Description

### FIELD OF THE INVENTION

This invention relates to coatings for ophthalmic implants. In particular, the present invention relates to the use of switchable tackiness coatings for ophthalmic implants.

### BACKGROUND OF THE INVENTION

The first generations of intraocular lenses were rigid implants. Modem IOLs are designed to be foldable so they can be inserted through smaller incisions. Foldable intraocular lens ("IOL") materials can generally be divided into three categories: silicone materials, hydrogel materials, and non-hydrogel acrylic materials. Many materials in each category are known. See, for example, Foldable Intraocular Lenses, Ed. Martin et al., Slack Incorporated, Thorofare, New Jersey (1993). Biocompatibility varies among different IOL materials within and among each category.

One measure of biocompatibility for an IOL can be the incidence of posterior capsule opacification ("PCO"). A number or factors may be involved in causing and/or controlling PCO. For example, the design and edge sharpness of an IOL may be a factor. See, Nagamoto et al., J. Cataract Refract. Surg., 23:866-872 (1997); and Nagata et al., Jpn. J. Ophthalmol., 40:397-403 (1996). See, also, U.S. Patent Nos. 5,549,670 and 5,693,094. Another factor appears to be the lens material itself. See, for example, Mandle, "Acrylic lenses cause less posterior capsule opacification than PMMA, silicone IOLs," Ocular Surgery News, Vol. 14. No. 15, p.23 (1996). See, also, Oshika, et al., "Two Year Clinical Study of a Soft Acrylic Intraocular Lens," J. Cataract. Refract. Surg., 22:104-109 (1996); and Ursell et al., "Relationship Between Intraocular Lens Biomaterials and Posterior Capsule Opacification," J. Cataract Refract. Surg., 24:352-360 (1998).

One method of addressing the PCO problem involves administering a pharmaceutical agent to the capsular bag area at the time of, or immediately after, extracapsular cataract extraction. See, for example, U.S. Patent Nos. 5,576,345 (pharmaceutical agent = the cytotoxic agent taxol or an ophthalmically acceptable derivative); 4,515,794; and 5,370,687. Alternatively, the pharmaceutical agent may be tethered to the surface of the IOL material. See, for example, U.S. Patent No. 4,918,165. The pharmaceutical agents are intended to kill or prevent the growth of proliferating cells that might cause PCO or "secondary cataracts." Yet another method involves the physical destruction or removal of lens epithelial cells. See, Saika et al., J. Cataract Refract. Surg., 23:1528-1531 (1997).

Other methods theorized for reducing the risk of PCO involve adhering the posterior capsule to the IOL at the time of implantation, as in U.S. Patent No. 5,002,571. According to the '571 patent, a non-biological glue or, preferably, a biological glue, such as fibrin, collagen, or mussel glue, is used to adhere the posterior lens capsule to the posterior surface of an IOL. The glue may be applied over the entire posterior surface of the IOL or just as an annulus around the outer perimeter of the posterior surface of the IOL.

Coating an IOL with a glue or tacky coating can make handling before and during surgery difficult. Ideally, an IOL or other ophthalmic implant for which a tacky surface is desirable at the target site in the eye would be tack-free during surgical handling and manipulation.

De Crevoisier et al., Science 285:1246-1249 (1999), disclose a fluorinated polymer coating material with switchable tackiness and wettability. The fluorinated polymer is a side-chain liquid crystalline copolymer obtained by radical copolymerization in butylacetate of 50 mole % of an acrylate monomer bearing a long perfluoroalkyl side chain (C₂H₄ - C₈F₁₇) and 50 % of a methacrylate monomer bearing a long alkyl chain (C₁₇H₃₅). Within a narrow temperature range centered at 35 °C, the copolymer undergoes a bulk transition between a highly ordered smectic phase and an isotropic phase. This transition from a hard, structured phase (nontacky) to a soft isotropic phase (tacky) modifies the copolymer's tackiness. According to de Crevoisier, et al., the ability to tune the adhesive and wettability properties of a material "opens interesting possibilities for many applications in which surface control is important, in areas as diverse as biotechnology, industrial coatings and cosmetics." The article lists anti-soil grips for tennis rackets or golf clubs as "everyday examples of switchable adhesive applications."

### SUMMARY OF THE INVENTION

The present invention relates to use of switchable tackiness coating compositions on ophthalmic implants. The coatings are not tacky at room temperature but become tacky when reaching the temperature of the eye. The coated implants are easily handled or manipulated by forceps, delivery injectors and other surgical tools at room temperature. After implantation, the coatings will become tacky, providing biological and clinical advantages such as reduced posterior capsule opacification for IOLs implanted in the capsular bag and enhanced fixation for intracorneal IOLs.

### DETAILED DESCRIPTION OF THE INVENTION

Unless otherwise indicated, all ingredient concentrations are listed as percent (w/w).

According to the present invention, ophthalmic implants are coated with a composition comprising
(i) a semifluorinated alkyl hydrocarbon monomer of the formula 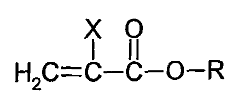 wherein
   X is H or CH₃; and
   R is C₆ - C₁₈ alkyl having at least 1/3 of the carbons substituted with fluorine, provided that the fluorinated carbons are consecutive carbons;
   and
(ii) an alkyl hydrocarbon monomer of the formula 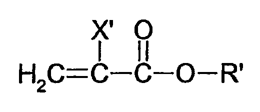 wherein
   X' is H or CH₃; and
   R' is C₆ - C₂₄ alkyl.

Preferred coating materials are those wherein
X and X' are not the same;
R is C₈ - C₁₆ wherein at least ½ of the carbons are substituted with fluorine, provided that the fluorinated carbons are consecutive carbons; and
R' is C₁₀ - C₂₀ alkyl.

The use of a combination of acrylate and methacrylate monomers is desirable for manipulating the coating T_{g}, however the use of both is not necessary. The thermodynamic stability of the liquid crystalline ("LC") phase and its position relative to the coating copolymer's T_{g} is important. The copolymer's T_{g} should be such that it does not override or freeze the LC phase; the copolymer's T_{g} should be below the temperature that the copolymer converts from a LC phase to an isotropic phase.

The identity of the monomers (i) and (ii) and their relative amounts are chosen so as to provide a LC phase transition temperature (from LC phase to an isotropic phase) at a temperature above room temperature (generally 18 - 25 C) and below the temperature of the eye (generally 34 - 37 C). The amount of monomer (i) relative to monomer (ii) should be about 1:3 - 3:1. Preferably, the coating consists essentially of the two monomers described above. Generally, the coating composition will contain about 33 - 66 % of monomer (i) and about 33 - 66 % of monomer (ii). Most preferred are coatings containing the two monomers in approximately a 1:1 ratio.

The coating compositions optionally include one or more ingredients selected from the group consisting of UV absorbers and blue-light blocking colorants, provided that they do not interfere with the ability of the coating to form a LC phase. The UV absorber and/or blue-light blocking colorant may be added either prior to polymerization or at the time the copolymer is dissolved to form a coating solution. If added prior to polymerization, the polymerization initiator should be chosen so that the UV absorber and/or blue-light blocking colorant do not significantly interfere with the polymerization.

Ultraviolet absorbing chromophores can be any compound which absorbs light having a wavelength shorter than about 400 nm, but does not absorb any substantial amount of visible light. It is preferred to use an ultraviolet absorbing compound that is copolymerizable with the monomers (i) and (ii) or the can be covalently bound to the coating/IOL substrate during the application of the coating to the substrate. Suitable copolymerizable ultraviolet absorbing compounds are the substituted 2-hydroxybenzophenones disclosed in U.S. Patent No. 4,304,895 and the 2-hydroxy-5-acryloxyphenyl-2H-benzotriazoles disclosed in U.S. Patent No. 4,528,311. The most preferred ultraviolet absorbing compound is 2-(3'-methallyl-2'-hydroxy-5'-methyl phenyl) benzotriazole.

If a UV-absorber is added prior to polymerization, it is unlikely that a UV polymerization initiator may be used. In such cases, the polymerization initiator will likely have to be either a thermal initiator or a blue-light initiator in order to avoid interference with the UV-absorber.

If a blue-light absorbing compound, e.g. a yellow dye, is included in the coating compositions of the present invention prior to polymerization, then the polymerization initiator will likely not be a blue-light photoinitiator. In the event the coating composition contains both a UV-absorber and a blue-light absorbing compound prior to polymerization, the polymerization initiator will likely be a thermal initiator. Preferably, the blue-light absorber is copolymerizable with the monomers (i) and (ii) or can be covalently bound to the coating/IOL substrate during the application of the coating. Suitable polymerizable blue-light blocking chromophores include those disclosed in U.S. Patent No. 5,470,932.

The coatings of the present invention may be applied to any ophthalmically acceptable implant material. Suitable materials include silicones and hydrogels. Preferably, the substrate material is a hydrophobic acrylic material. As used herein, "hydrophobic acrylic material" means a (meth)acrylic material having an equilibrium water content of not more than about 5 % by weight as determined gravimetrically in deionized water at ambient conditions. Examples of such (meth)acrylic materials are disclosed in the following U.S. Patent Nos.: 5,290,892; 5,693,095; and 5,331,073.

The coatings of the present invention may be applied to any ophthalmic implant including, but not limited to, IOLs designed for anterior or posterior chamber implantation; intracorneal lenses; keratoprostheses; and corneal inlays or rings.

In the case where the ophthalmic implant is an IOL, the coatings of the present invention may be used in conjunction with substrate materials that are "hard" (that is, materials that are inserted in an unfolded state) or "foldable" or "soft" (that is, materials that are capable of being inserted in a folded or compressed state). The coating may be applied to the portion of the IOL material constituting the optic's entire posterior surface or to only a peripheral band of the posterior surface. Additionally, the optic's anterior surface or a peripheral band of the anterior surface may be coated.

In order to prepare the ophthalmic implant to be coated so that it is capable of receiving the coating, it may be necessary or desirable to expose the surface to be coated to a reactive plasma gas prior to applying the coating composition of the present invention. Suitable reactive plasma gases include oxidizing gases, such as oxygen gas. A suitable plasma chamber is the p²CIM B-Series plasma chamber made by Advanced Plasma Systems, Inc.

The coating composition of this invention is applied using known coating techniques. For example, the coatings can be applied by first polymerizing a mixture of monomers (I) and (ii) using a thermal initiator, such as benzoyl peroxide or photoinitiator. After polymerization, the coating material is dissolved in an organic solvent, such as butyl acetate, to form a coating solution. The coating solution is then applied to the ophthalmic implant using conventional dip- or spin-coating techniques. Alternatively, the monomers (i) and (ii) can be loaded into a plasma chamber and deposited onto the surface of an ophthalmic implant using conventional plasma deposition techniques.

Regardless of the method of applying the coating to the ophthalmic article, the thickness of the coating should be at least about 0.3 µm, preferably at least about 1 - 2 µm, but may be thicker provided that the coating remains transparent.

### Example

A reaction flask containing a magnetic stir bar was charged with 1.508 g heptadecylfluorodecyl acrylate, 0.967 g octadecyl methacrylate and 2.547 g butyl acetate. Nitrogen was bubbled through the mixture with stirring for 15 min, then 0.023 g of benzoyl peroxide was added with continued nitrogen bubbling. The flask was closed with a glass stopper and immersed in a 70 °C oil bath for 6.5 hr. The reaction product was precipitated directly in 300 mL of methanol, filtered, rinsed with methanol and dried for 24 hrs under vacuum. Yield: 1.524g (62%) of a white solid. The solid became fluid just about body temperature as a small piece would become tacky on warming slightly. The cooled material was non-tacky. Examination between cross polarizers on the microscope revealed a LC texture on shearing. The resulting polymer was soluble in butyl acetate. Differential scanning calorimetry revealed a phase transition at 31 °C on cooling, 39 °C on heating.

## Claims

1. A coated ophthalmic implant comprising a substrate material and a coating material that is substantially tack-free at room temperature but tacky at body temperature, wherein the coating material comprises
(i) a semifluorinated alkyl hydrocarbon monomer of the formula 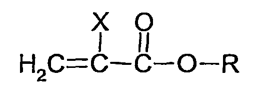 wherein
X is H or CH₃; and
R is C₆ - C₁₈ alkyl having at least 1/3 of the carbons substituted with fluorine, provided that the fluorinated carbons are consecutive carbons;
and
(ii) an alkyl hydrocarbon monomer of the formula 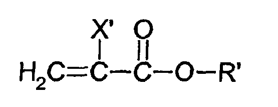 wherein
X' is H or CH₃; and
R' is C₆ - C₂₄ alkyl.

2. The coated ophthalmic implant of Claim 1 wherein
X and X' are not the same;
R is C₈ - C₁₆ wherein at least ½ of the carbons are substituted with fluorine, provided that the fluorinated carbons are consecutive carbons; and
R' is C₁₀ - C₂₀ alkyl.

3. The coated ophthalmic implant of Claim 1 wherein the amount of monomer (i) relative to the amount of monomer (ii) is about 1:3 - 3:1 (w/w).

4. The coated ophthalmic implant of Claim 1 wherein monomer (i) and monomer (ii) are present in a 1:1 ratio.

5. The coated ophthalmic implant of Claim 1 wherein the coating further comprises one or more ingredients selected from the group consisting of UV absorbers and blue-light blocking colorants.

6. The coated ophthalmic implant of Claim 1 wherein the substrate material is selected from the group consisting of silicone; hydrogel; and hydrophobic acrylic materials.

7. The coated ophthalmic implant of Claim 1 wherein the implant is selected from the group consisting of intraocular lenses designed for anterior or posterior chamber implantation; intracorneal lenses; keratoprostheses; and corneal inlays or rings.

8. The coated ophthalmic implant of Claim 1 wherein the coating is at least about 0.3 µm thick.

9. The coated ophthalmic implant of Claim 8 wherein the coating is at least about 1 µm thick.

## Patentansprüche

1. Beschichtetes Augenimplantat, umfassend ein Substratmaterial und ein Beschichtungsmaterial, das bei Raumtemperatur im wesentlichen klebefrei, bei Körpertemperatur aber klebrig ist, wobei das Beschichtungsmaterial
(i) ein halb-fluoriertes Alkylkohlenwasserstoffmonomer der Formel 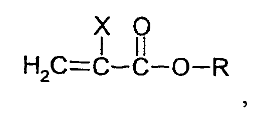 worin
X H oder CH₃ ist; und
R C₆-C₁₈-Alkyl ist, bei dem mindestens 1/3 der Kohlenstoffe mit Fluor substituiert ist, vorausgesetzt, daß die fluorierten Kohlenstoffe aufeinanderfolgende Kohlenstoffe sind;
und
(ii) ein Alkylkohlenwasserstoffmonomer der Formel 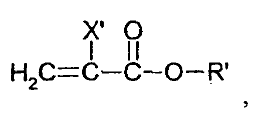 worin
X' H oder CH₃ ist; und
R' C₆-C₂₄-Alkyl ist,
umfaßt.

2. Beschichtetes Augenimplantat nach Anspruch 1, wobei
X und X' nicht dieselben sind;
R C₈-C₁₆ ist, bei dem mindestens 1/2 der Kohlenstoffe mit Fluor substituiert ist, vorausgesetzt, daß die fluorierten Kohlenstoffe aufeinanderfolgende Kohlenstoffe sind; und
R' C₁₀-C₂₀-Alkyl ist.

3. Beschichtetes Augenimplantat nach Anspruch 1, wobei die Menge an Monomer (i), bezogen auf die Menge von Monomer (ii), etwa 1 : 3 - 3 : 1 (Gew./Gew.) beträgt.

4. Beschichtetes Augenimplantat nach Anspruch 1, wobei Monomer (i) und Monomer (ii) in einem Verhältnis von 1 : 1 vorliegen.

5. Beschichtetes Augenimplantat nach Anspruch 1, wobei die Beschichtung ferner einen oder mehrere Inhaltsstoffe, ausgewählt aus der Gruppe, besehend aus UV-Absorbern und Blaulicht blockierenden Färbemitteln, umfaßt.

6. Beschichtetes Augenimplantat nach Anspruch 1, wobei das Substratmaterial aus der Gruppe, besehend aus Silikon; Hydrogel und hydrophoben Acrylmaterialien, ausgewählt ist.

7. Beschichtetes Augenimplantat nach Anspruch 1, wobei das Implantat aus der Gruppe, besehend aus intraokularen Linsen, gestaltet für die Implantation in die vordere oder hintere Kammer; intrakornealen Linsen, Hornhautprothesen und kornealen Inlays oder Ringen, ausgewählt ist.

8. Beschichtetes Augenimplantat nach Anspruch 1, wobei die Beschichtung mindestens etwa 0,3 µm dick ist.

9. Beschichtetes Augenimplantat nach Anspruch 8, wobei die Beschichtung mindestens etwa 1 µm dick ist.

## Revendications

1. Implant ophtalmique pourvu d'un revêtement comprenant un matériau de substrat et un matériau de revêtement qui est sensiblement non collant à température ambiante mais collant à température corporelle, dans lequel le matériau de revêtement comprend :
(i) un monomère d'hydrocarbure d'alkyle semi fluoré de formule 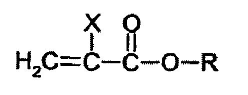 dans lequel
X est H ou CH₃ ; et
R est un alkyle en C₆ - C₁₈ possédant au moins un tiers des atomes de carbone substitués par du fluor, à condition que les atomes de carbone fluorés soient des atomes de carbone consécutifs ;
et
(ii) un monomère d'hydrocarbure d'alkyle de formule 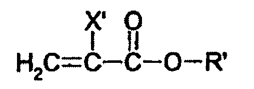 dans lequel
X' est H ou CH₃ ; et
R' est un alkyle en C₆ - C₂₄.

2. Implant ophtalmique pourvu d'un revêtement de la revendication 1, dans lequel
X et X' sont différents ;
R est un alkyle en C₈ - C₁₈ dans lequel au moins la moitié des atomes de carbone sont substitués par du fluor, à condition que les atomes de carbone fluorés soient des atomes de carbone consécutifs ; et
R' est un alkyle en C₁₀ -C₂₀.

3. Implant ophtalmique pourvu d'un revêtement de la revendication 1, dans lequel la quantité du monomère (i) par rapport à la quantité du monomère (ii) est d'environ 1:3 - 3:1 (en poids).

4. Implant ophtalmique pourvu d'un revêtement de la revendication 1, dans lequel le monomère (i) et le monomère (ii) sont présents avec un rapport 1:1.

5. Implant ophtalmique pourvu d'un revêtement de la revendication 1, dans lequel le revêtement comprend en outre un ou plusieurs ingrédients choisis dans le groupe constitué d'absorbeurs UV et de colorants arrêtant la lumière bleue.

6. Implant ophtalmique pourvu d'un revêtement de la revendication 1, dans lequel le matériau de substrat est choisi dans le groupe constitué de silicone ; d'hydrogel ; et de matériaux acryliques hydrophobes.

7. Implant ophtalmique pourvu d'un revêtement de la revendication 1, dans lequel l'implant est choisi dans le groupe constitué des lentilles intraoculaires conçues pour une implantation dans la chambre postérieure ou antérieure ; des lentilles intra cornéennes ; des kératoprothèses ; et des greffes encastrées cornéennes ou anneaux cornéens.

8. Implant ophtalmique pourvu d'un revêtement de la revendication 1, dans lequel le revêtement présente une épaisseur d'au moins 0,3 µm.

9. Implant ophtalmique pourvu d'un revêtement de la revendication 8, dans lequel le revêtement présente une épaisseur d'au moins 1 µm.
